# EUROPEAN PATENT SPECIFICATION

(11) **EP 4 331 650 B1**
(45) Date of publication and mention of the grant of the patent: **04.11.2026**
(21) Application number: 23215448.4
(22) Date of filing: 20.01.2021
(51) Int. Cl.: A61B 5/087, A61M 16/00

(54) **A DEVICE FOR A RESPIRATION ARRANGEMENT**
VORRICHTUNG FÜR EINE BEATMUNGSANORDNUNG
DISPOSITIF POUR UN AGENCEMENT DE RESPIRATION

(30) Priority: 23.01.2020 SE 2050064
(43) Date of publication of application: 06.03.2024
(62) Document number(s) of the earlier application(s) in accordance with Art. 76 EPC: 21702392.8 / 4 093 276
(73) Proprietor: Monivent AB, 417 55 Göteborg (SE)
(72) Inventor: LÖFGREN, Mikael, 431 69 Mölndal (SE); STÅLNACKE, Antti, 445 56 Surte (SE)
(74) Representative: Kransell & Wennborg KB

(56) References cited:
- US-A1- 2010 229 865
- US-B1- 6 224 560

## Description

### Field of the Invention

The present inventive concept relates to a device for a respiration arrangement, typically for breathing support to patients, preferably infants. The present inventive concept also relates to a respiration arrangement comprising a patient interface such as e.g. a face mask, an air/gas source, and the device.

### Background of the Invention

At birth, an infant may require immediate support in order to initiate breathing or support to sustain breathing. This may be the case due to apnea, airway obstruction, respiratory distress syndrome or when the infant is prematurely born due to their immature respiratory system. Sometimes the infant is in need of immediate breathing support directly after birth, which is normally carried out by manually pumping air into the lungs of the infant. Breathing support, or manual ventilation, may also be performed in other situations, and can be conducted on any patient regardless of his or her age.

A respiration device, or respiration arrangement, typically includes a patient interface such as e.g. a face mask to fit over the nose and mouth of the patient, an endotracheal tube, a laryngeal mask or nasal prongs, an air source (or another gas source, e.g. oxygen), and an air pathway providing the air from the air source to the face mask. The air source may e.g. be a resuscitation bag/bellow. In many situations, there is a need for monitoring the patient's respiration, or measuring the ventilation of a patient not breathing. Thus, there is a need to know the amount of air fed to the patient. The monitoring is typically performed by a monitoring arrangement arranged along the air pathway, including a flow sensor using e.g. a pressure sensor, such as a differential pressure sensor, for determining the flow of air, and a display disclosing information of the respiration. An example of such resuscitation arrangement is disclosed in WO 2015/167388 A1.

Flow sensors are typically based on the Venturi principle, which means that the pressure drop associated with a flow constriction in the air pathway is measured. An algorithm transforming the differential pressure to a flow is typically carried out by the means of a processor or CPU and a memory. In order to achieve a high level of accuracy of the flow measurement, the flow sensor needs to be carefully designed with regards to e.g. the geometry of the flow constriction. In US6224560B1, an example of a flow measurement is shown. US6224560B1 discloses a flow restrictor applied to a respiration-channeling device such as a tracheostomy tube, a mouthpiece, or a face mask. The flow restrictor creates a pressure differential which varies with volume flow rate. A pressure reading is taken on either side of the flow restrictor in the respiration-channeling device and from this, a volume flow rate is determined. A data acquisition device is used to derive further pulmonary function parameters from the volume flow rate.

Due to the risk of cross-contamination between patients, single-use units are preferred for those parts being subject to be contaminated by the patient. Thus, there is drive to keep components costs as low as possible. However, as accuracy of the flow measurement is of high importance, there is often a conflict between costs and accuracy. There is thus a need in the industry for a respiration arrangement, and associated parts, which better balance the cost and measurement accuracy.

### Summary

An object of the inventive concept is to overcome the above problems, and to provide a device which, at least to some extent, is less complex than prior art solutions, while still enabling a sufficient measurement accuracy. This, and other objects, which will become apparent in the following, are accomplished by means of a device for a respiration arrangement, and a respiration arrangement comprising such device as defined in the accompanying claims.

The present inventive concept is based on the insight that a more accurate flow measurement can be performed by controlling the fluid flow to, and/or in, the flow constriction in the air pathway when fluid flows from the air/gas source to the patient interface (e.g. a face mask, endotracheal tube, laryngeal mask or nasal prongs) or in the opposite direction, from the patient interface to the air/gas source. For example, the fluid may be controlled by a flow guiding element guiding flow towards the flow constriction and/or by providing the flow constriction with a laminar flow section.

According to an unclaimed aspect, a device for a respiration arrangement is provided. The device comprises:
- a conduit having a first opening connectable to an air/gas source such as a resuscitation bag, and a second opening connectable to a patient interface, such that a fluid pathway along a longitudinal direction of the conduit is established from the first opening to the second opening;
- a flow constriction, arranged in the conduit which upon fluid flow through the conduit results in a pressure difference over the flow constriction, wherein the device further comprises at least one flow guiding element arranged in the conduit between the first opening and the flow constriction, and configured to guide fluid flow to the flow constriction.

Hereby, any accuracy problem originating from the flow measurement with regards to asymmetrical flow constrictions, as e.g. flow constrictions not being centrally arranged in the conduit, or flow constrictions causing an undesired turbulence of the flow, can be avoided or at least reduced. Thus, the flow guiding element enables accurate measurement independently of the orientation of the incoming fluid flow as it guides the fluid flow towards the flow constriction. Furthermore, as the flow guiding element occupies space inside the conduit, it contributes in reducing undesired dead space inside the device, thereby improving the respiration procedure.

According to a first aspect of the present inventive concept, a device for a respiration arrangement in accordance with claim 1 is provided.

It should be noted that the device may e.g. be an adapter, such as an airway adapter, which is detachably arranged to the patient interface (e.g. a face mask) of the respiration arrangement. As an alternative, the device may form a part of, or comprise, an integrated patient interface, e.g. in the form of a face mask, endotracheal tube, laryngeal mask or nasal prongs, attached to the conduit at an end portion comprising the second opening. The device may also be referred to as a respiration device, a conduit arrangement, or an air transportation arrangement. For embodiments in which the patient interface is a face mask, the face mask may e.g. be a soft mask and the device forming part of a hard mask, or an adapter, connectable to the soft mask.

In the following, the patient interface is mainly referred to as a face mask, but it should be understood that it may as well be another patient interface, such as e.g. endotracheal tube, a laryngeal mask or nasal prongs. Thus, when stating that the second opening is connectable to a face mask, it may as well be connectable to another patient interface, such as the endotracheal tube, the laryngeal mask or the nasal prongs. The face mask, the endotracheal tube, the laryngeal mask and the nasal prongs may be referred to as patient interfaces, as they serve as an interface between the second opening of the conduit of the device, and the patient.

It should be understood that the conduit may provide an air/gas pathway in both directions, i.e. both from the air/gas source to the face mask via the first opening, the conduit and the second opening in that order, and from the face mask to the air/gas source via the second opening, the conduit and the first opening in that order. In the former case, the first opening acts as a conduit inlet and the second opening acts as a conduit outlet, and the flow constriction is arranged downstream of the inlet. In the other case, when the fluid flows from the face mask to the air/gas source, typically as the patient exhales, the second opening acts as conduit inlet and the first opening acts as conduit outlet. The exhaled air/gas is then typically ventilated out from the respiration arrangement. Hence, the flow guiding element is, upon fluid flow from the first opening to the second opening, arranged and configured to guide fluid flow towards, or to, the flow constriction. When fluid is flowing in the opposite direction, i.e. from the second opening to the first opening, fluid is transported through the flow guiding element in a reverse direction, i.e. from the flow constriction towards the first opening. Thus, the flow guiding element may be arranged and configured to transport the flow through the flow guiding element. In other words, the flow guiding element may comprise a channel or fluid passage enabling fluid flow between the first opening and the flow constriction, and between the flow constriction and the first opening. According to at least one example embodiment, the conduit comprises the conduit walls surrounding the air/gas pathway. That is, the conduit walls are formed of a separate element (or separate part) compared to the flow guiding element, or stated differently, the conduit walls and the flow guiding elements are separate elements (or separate parts). For example, the conduit with its conduit walls may be physically separated from the flow guiding element prior to insertion of the latter into the conduit. Hereby, the conduit and its conduit walls may be manufactured separately to the flow guiding element. After insertion of the flow guiding element into the conduit, the flow guiding element is typically in contact with the conduit walls, e.g. by a press-fit or by some type of welding or other contacting means. As the conduit and its conduit walls form a separate element (or part) compared to the flow guiding element, the flow guiding element may be detachably arranged in the conduit.

Furthermore, the flow constriction which is arranged in the conduit to, upon fluid flow through the conduit, result in a pressure difference over the flow constriction, is designed so that the pressure difference regardless of the direction of the fluid flow (from air/gas source to face mask, or vice versa) can be used for e.g. flow measurement. That is, the flow of air/gas provided to the face mask from the air/gas source can be measured, as well as the flow of air/gas from the face mask, caused by exhalation of the patient, towards the air/gas source. By measuring the flow in both directions, the amount of leakage in the system can be determined.

According to at least one example embodiment, the flow guiding element is detachably arranged to the conduit. Thus, the flow guiding element can be removed upon will from the conduit via the first opening e.g. for maintenance or adaptation reasons, and then subsequently be refitted into the conduit. That the flow guiding element is detachably arranged to the conduit may alternatively, or additionality, imply that the flow guiding element may be removed from the conduit after use and e.g. be recycled or otherwise re-used. Thus, the flow guiding element is typically attached to the conduit, e.g. by being press-fitted into the conduit and/or welded or otherwise connected to the conduit walls, but may be de-attached from the conduit by simply pulling the flow guiding element out of the conduit possibly subsequent to removing any welds or other connector (if present) of the flow guiding element to the conduit walls.

According to at least one example embodiment the air/gas source is a resuscitation bag or a T-piece resuscitator. Alternatively, air/gas source may be an air source or another gas source, such as e.g. an oxygen source. Thus, the conduit of the device is preferably at the end portion comprising the first opening arranged and configured to be connected to the air/gas source, such as a resuscitation bag.

According to at least one example embodiment, the device comprises at least one pressure connecting port arranged in pressurized communication with fluid between the flow constriction and the first opening, the pressure connecting port being connectable to a pressure sensor for measuring fluid pressure.

According to at least one example embodiment, the device comprises at least one pressure connecting port arranged in pressurized communication with fluid between the flow constriction and the first opening, the pressure connecting port being connectable to a pressure sensor for measuring fluid pressure, and wherein the pressure connecting port is arranged in the longitudinal direction of the conduit.

Hereby, the at least one pressure connecting port and the flow constriction have fluid pathways arranged in the same direction (the longitudinal direction of the conduit), which facilitate manufacturing (e.g. by injection molding) of the device. Moreover, by such orientation of the at least one pressure connecting port, a filter, such as a bacterial filter, may easily be inserted into the device, and further into the pressure connecting port, via the first opening.

**It** should be understood that when stating that the at least one pressure connecting port is arranged in the longitudinal direction of the conduit, the cross section of an inlet of the at least one pressure connecting port is arranged perpendicular to the longitudinal direction. Or stated differently, the at least one pressure connecting port comprises an inlet facing a geometrical plane being perpendicular to the longitudinal direction. However, it should be noted that the inlet of the at least one pressure connecting port is not arranged in the conduit to receive any fluid flow, but rather to act as a port for enabling fluid communication enabling measurement of the pressure of the fluid between the flow constriction and the first opening (i.e. upstream of the flow constriction when fluid flows from the first opening to the second opening). Stated differently, the at least one pressure connecting port comprises an elongated fluid cavity having a longitudinal axis being parallel to the longitudinal direction of the conduit.

According to at least one example embodiment, the flow guiding element is configured to guide fluid flow away from the pressure connecting port. For example, the flow guiding element may comprise a flow passage guiding flow away from the pressure connecting port, towards the flow constriction.

The at least one pressure connecting port may according to at least one example embodiment simply be referred to as a pressure port.

According to at least one example embodiment, the at least one pressure connecting port is at least partly arranged in parallel to the flow constriction. Hereby, the device may be made more compact.

According to at least one example embodiment, the flow constriction at least partly comprises a laminar flow section having a plurality of elongated channels arranged in the longitudinal direction of the conduit such that the at least one pressure connecting port and the plurality of elongated channels are arranged at least partly in parallel.

Thus, manufacturing of the device is facilitated owing to the coherent direction of the fluid channels of the at least one pressure connecting port and the elongated channels. Moreover, by arranging the at least one pressure connecting port and the plurality of elongated channels at least partly in parallel, the device may be made more compact, as a cross section of the conduit perpendicular to the longitudinal direction, at the flow constriction, comprises both the at least one pressure connecting port and the elongated channels. In other words, at least a part of the pressure connecting port is sharing the same conduit space as the flow constriction. By such configuration, the flow constriction is typically asymmetrical arranged in the conduit, and the flow guiding element may serve its purpose by directing the fluid flow towards the flow constriction. Without such flow guiding element, fluid would be able to flow into the pressure connecting port and thereby reduce the measurement accuracy.

It should be understood that each channel in the plurality of elongated channels has a main direction in the longitudinal direction. The elongated channels may be referred to as elongated holes or tubes. The number, and the length of the channels, are typically adapted based on the expected fluid flow through the flow constriction with the purpose to achieve a laminar flow instead of a turbulent flow, as this improves the measurement accuracy. How to design the laminar flow section in order to achieve a laminar flow is known to the skilled person. It should also be noted that as the laminar flow condition is at least partly dependent on the fluid flow, where a high fluid flow increases the risk of turbulence occurrence, fluid flow in at least a part of the elongated channels may still at times be turbulent. According to at least one example embodiment, the laminar flow section may simply be referred to as a flow section.

Typically, the laminar flow section constitutes at least a portion of the flow constriction, and thus fully accommodate the passageway in the flow constriction, in at least a part along the longitudinal direction of the conduit. According to at least one example embodiment, the laminar flow section is the flow constriction of the device.

According to at least one example embodiment, the laminar flow section comprises a net having a plurality of holes, instead of the elongated channels. Thus, such net serves the same purpose as the elongated channels, i.e. to provide a laminar flow.

It should be understood that the purpose of the laminar flow section is to provide a relatively large contact surface between the internal walls of the elongated channels and the fluid, which results in a more linear relation between fluid flow and pressure drop, thereby improving measurement accuracy.

The device of the first aspect of the inventive concept comprises:
- a conduit having a first opening connectable to an air/gas source such as a resuscitation bag, and a second opening connectable to a patient interface, such that a fluid pathway along a longitudinal direction of the conduit is established from the first opening to the second opening;
- a flow constriction arranged in the conduit which upon fluid flow through the conduit results in a pressure difference over the flow constriction,
wherein the flow constriction at least partly comprises a laminar flow section, wherein the device further comprises at least one pressure connecting port arranged in pressurized communication with fluid between the flow constriction and the first opening, the pressure connecting port being connectable to a pressure sensor for measuring fluid pressure, and wherein the pressure connecting port is arranged in the longitudinal direction of the conduit.

Thus, the first aspect of the inventive concept is very similar to the earlier mentioned unclaimed aspect, but instead of (in its broadest concept) providing a flow guiding element guiding the flow towards the flow constriction, the flow inside the flow constriction is controlled by a laminar flow section, and the pressure connecting port. Thus, effects and features related to the pressure connecting port and laminar flow section of this first aspect of the present inventive concept are largely analogous to those described above in connection with the unclaimed aspect.

According to at least one example embodiment, the device of the first aspect of the inventive concept comprises at least one flow guiding element as previously described.

Hereafter, embodiments related to the first aspect of the inventive concept are described in detail.

According to at least one example embodiment, the flow guiding element comprises:
- a shielding portion arranged in the conduit to cover an inlet of the pressure connecting port,
- a fluid passage enabling fluid flow between the first opening and the flow constriction, and
- at least one pressure conduit arranged to provide a fluid channel between the fluid passage and the pressure connecting port.

Hereby, fluid flow into, or through, the pressure connecting port can be avoided by the shielding portion, while still providing a fluid passage, which is guided towards the flow constriction (typically towards the laminar flow section). Thus, the pressure connecting port can be in fluid communication, without receiving any fluid flow, with fluid present between the first opening and the flow constriction via the at least one pressure conduit (i.e. upstream conditions of the flow constriction when fluid flows through the device from the first opening to the second opening). Thus, the pressure of the fluid can be measured by a pressure sensor connected to the pressure connecting port.

According to at least one example embodiment, the fluid passage is shaped to enable a minimum flow resistance, e.g. by having a crescent shaped cross section perpendicular to the longitudinal direction of the conduit. Hereby, the breathing support to the patient is improved. Thus, a majority of the cross section of the flow guiding element not used as shielding portion, can be used for the fluid passage.

According to at least one example embodiment, the at least one pressure conduit is arranged in proximity to an end portion of the fluid passage in order to minimize influence of potential fluid turbulence. According to at least one example embodiment, the flow guiding element comprises at least two pressure conduits in order to decrease measurement errors.

According to the first aspect of the present inventive concept, the pressure connecting port is a first pressure connecting port, and the device further comprises a second pressure connecting port arranged in pressurized communication with fluid between the flow constriction and the second opening, such that a differential pressure sensor can be connected to the first and second pressure connecting ports for measuring the differential pressure over the flow constriction.

Hereby, the flow through the flow constriction can be measured by e.g. a flow sensor based on the Venturi principle or Bernoulli equation, which means that the pressure drops associated with a flow constriction in the air pathway is measured. An algorithm transforming the differential pressure to a flow is may be carried out by the means of a processor or CPU and a memory.

According to at least one example embodiment, the second pressure connecting port is arranged in the longitudinal direction of the conduit. Thus, a cross section of an inlet of the second pressure connecting port is arranged perpendicular to the longitudinal direction. Stated differently, the second pressure connecting port comprises an elongated fluid cavity having a longitudinal axis being parallel to the longitudinal direction of the conduit.

According to the first aspect of the present inventive concept, the device comprises a pressure sensor connecting portion arranged between the first opening and the second opening, typically close to the flow constriction. The pressure sensor connecting portion is connectable to a pressure sensor unit. Typically, the pressure sensor connecting portion comprises a tube-shaped portion extending from the conduit perpendicular to the longitudinal direction. Moreover, the first and second pressure connecting ports are arranged in the device to have respective openings (which may be referred to as outlets of the pressure connecting ports) in the pressure sensor connecting portion, each of the openings having a cross section parallel to the longitudinal direction. Such openings are thus arranged in the opposite end to the opening of the respective pressure connecting port which have a cross section perpendicular to the longitudinal direction of the conduit (i.e. the openings which were referred to as inlets of the pressure connecting ports).That is, each one of the first and second pressure connecting ports may have an L-shaped channel.

According to at least one example embodiment, fluid flow in the pressure connecting ports is minimized, at least fluid flow through the respective pressure connecting port. Hereby, any contamination which may enter into the device will not propagate into a connected pressure sensor.

According to at least one example embodiment, the flow guiding element is a first flow guiding element and the device further comprises a second flow guiding element arranged in the conduit between the flow constriction and the second opening, and configured to guide fluid flow away from the second pressure connecting port.

Thus, when fluid flows from the second opening to the first opening of the conduit (i.e. when the patient exhales and fluid flows in a direction from the patient interface towards the air/gas source), the fluid is guided towards the flow constriction in a similar manner as described above in relation to the first flow guiding element. Hence, the second flow guiding element is, upon fluid flow from the second opening to the first opening, arranged and configured to guide fluid flow towards, or to, the flow constriction. When fluid is flowing in the opposite direction, i.e. from the first opening to the second opening, fluid is transported through the second flow guiding element in a reverse direction, i.e. from the flow constriction towards the second opening. Hereby, any accuracy problem originating from the flow measurement with regards to asymmetrical flow constrictions, as e.g. flow constrictions not being centrally arranged in the conduit, or flow constrictions causing an undesired turbulence of the flow, can be avoided or at least reduced. Thus, the second flow guiding element enables accurate measurement independently of the orientation of the incoming fluid flow as it guides the fluid flow towards the flow constriction, and away from the second pressure connecting port. Furthermore, as the second flow guiding element occupies space inside the conduit, it contributes in reducing undesired dead space inside the device, thereby improving the respiration procedure. This is especially important for embodiments having a laminar flow section as the large contact area between inner walls of the laminar flow section and fluid flow typically leads to an increased dead space. Thus, the dead space reduction owing to the flow guiding element(s) counteract the increased dead space resulting from the laminar flow section.

According to at least one example embodiment, the second flow guiding element acts as a protector preventing any liquid (saliva, vomit) expectorated from the patient to reach further into the conduit, thereby preventing contaminations to propagate further into the device. This is especially important for embodiments having a laminar flow section as the narrow passageway(s) otherwise could be clogged.

According to at least one example embodiment, the second flow guiding element comprises
- a shielding portion arranged in the conduit to cover an inlet of the second pressure connecting port,
- a fluid passage enabling fluid flow between the second opening and the flow constriction,
- at least one pressure conduit arranged to provide a fluid channel between the fluid passage and the second pressure connecting port, and
- a protecting portion arranged in the conduit to prevent any liquid expectorated from the patient to reach further into the conduit.

Thus, the second flow guiding element also serve to protect the device from contamination from the patient. That is, the protecting portion of the second flow guiding element prevents liquid, such as saliva or vomit, from the patient to reach into the device beyond the second flow guiding element. This is especially important for embodiments have a laminar flow section due to the narrow passageway(s).

The second flow guiding element is, correspondingly to the first flow guiding element, configured and arranged to prevent fluid flow into, or through, the second pressure connecting port by the shielding portion, while still providing a fluid passage, which is guided towards the flow constriction (typically towards the laminar flow section). Thus, the second pressure connecting port can be in fluid communication, without receiving any fluid flow, with fluid present between the second opening and the flow constriction via the at least one pressure conduit. Thus, the pressure of the fluid can be measured by a pressure sensor connected to the second pressure connecting port.

According to at least one example embodiment, the fluid passage is arranged between internal walls of the conduit and the protecting portion. Preferably, the fluid passage of the second flow guiding element is arranged in proximity to the internal walls of the conduit. Correspondingly, the protecting portion is preferably arranged closer to the center of the conduit compared to the fluid passage in order to efficiently hinder any liquid expectorated from the patient.

According to at least one example embodiment, the device further comprises a patient interface, such as e.g. a face mask, connected to the conduit at and end portion comprising the second opening.

That is, the conduit may comprise a first end portion arranged at the first opening, and a second end portion arranged at the second opening. Thus, the patient interface may be connected to, such as detachably connected to, or integrated with, the second end portion of the conduit such that the second opening lead into the void of the patient interface, such as e.g. the void of a face mask.

As already stated above, instead of a face mask, another patient interface such as e.g. an endotracheal tube, a laryngeal mask or nasal prongs may be chosen.

According to at least one example embodiment, the device is a single-use component, and is to be disposed after usage. Hereby, contamination between patients can be minimized.

According to at least one example embodiment, the device is injection molded. This provides for a suitable and cheap way of producing the device.

That is, the device is manufactured by an injection molding process. This is especially advantageous with regards to the specific geometry needed for the laminar flow section, such as the production of the plurality of channels. Specifically, the orientation of the pressure connecting ports, at least the respective portion arranged to face the first and second opening of the conduit, respectively, and the direction of the plurality of channels in the laminar flow section are aligned in the longitudinal direction of the conduit during the injection molding process.

According to at least one example embodiment, the device further comprises a pressure sensor unit detachably arranged to the conduit for measuring pressure upstream and/or downstream of the flow constriction.

Thus, the pressure sensor unit may measure the pressure difference over the flow constriction, i.e. at a location between the first opening and the flow constriction, and a location between the flow constriction and the second opening. Thus, the pressure sensor unit typically comprises channels enabling connections to the first and second pressure connecting ports, in order to transport the fluid pressure inside of the pressure sensor unit to a pressure sensor.

Advantageously, the pressure sensor unit is detachably arranged to the conduit via the pressure sensor connecting portion.

According to at least one example embodiment, the pressure sensor unit is not a part of the device, and is thus considered as a separate component to the device. For example, the device (without the pressure sensor unit) may be a single-use component (i.e. disposable) while the pressure sensor unit may be re-used.

According to at least one example embodiment, the pressure sensor unit may e.g. comprise a differential pressure sensor for measuring the differential pressure over the flow constriction by being in pressurized communication with the first and second pressure ports. The pressure sensor unit may alternatively or additionally comprise a gauge pressure sensor to assess the (positive) pressure used during ventilation. According to at least one example embodiment the gauge pressure sensor is realized using an absolute pressure sensor comparing the (ambient) pressure measured before ventilation, with the pressure during ventilation and then calculating the gauge pressure by subtracting these values. This allows for using only one (cheap yet accurate) barometric sensor to assess both ambient pressure and gauge pressure. According to at least one example embodiment, the pressure sensor unit comprises a pressure sensor preventing fluid flow through the pressure sensor by e.g. a membrane. Thus, the pressure sensor may be a membrane-based pressure sensor.

Moreover, the pressure sensor unit may comprise a battery, a CPU or processor, a memory, a wireless communication means, such as e.g. a Bluetooth module. The pressure sensor unit may furthermore comprise a housing holding all of the previously mentioned components of the pressure sensor unit.

As mentioned above, the device is configured and arranged so that there will be no fluid flow through the respective first and second pressure connecting ports. Thus, there will be no fluid flow in the pressure sensor unit when being attached to the conduit of the device. Typically, this would lead to poor zero level stability of pressure measurement. However, the laminar flow section mentioned above in accordance with an embodiment of the invention, counteracts this drawback, resulting in a satisfying accuracy of the flow measurement.

According to at least one example embodiment, the device comprises one or more filters arranged in the first and/or second pressure connecting ports. Hereby, contamination further into the pressure sensor unit is further prevented.

According to a second aspect of the present inventive concept, a respiration arrangement for breathing support to a patient, preferably an infant, is provided. The respiration arrangement comprises:
- a patient interface, such as a face mask adapted to fit over the mouth and nose of the patient,
- an air/ gas source providing air or gas to the patient interface, and
- a device according to the first aspect of the inventive concept.

Effects and features related to the second aspect of the present inventive concept are largely analogous to those described above in connection with the first aspect of the inventive concept. For example, the air/gas source may e.g. be a resuscitation bag, and the patient interface may, instead of a face mask, be an endotracheal tube, a laryngeal mask or nasal prongs.

According to at least one example embodiment, the patient interface is a face mask which is connected to, such as detachably connected to, or integrated with, the second end portion of the conduit such that the second opening lead into the void of the face mask.

Further features of, and advantages with, the present inventive concept will become apparent when studying the appended claims and the following description. The skilled person realize that different features of the present inventive concept may be combined to create embodiments other than those described in the following, without departing from the scope of the present inventive concept.

### Brief Description of the Drawings

These and other aspects of the present inventive concept will now be described in more detail, with reference to the appended drawings showing an example embodiment of the inventive concept, wherein:
Fig. 1 schematically illustrates a respiration arrangement for breathing support to a patient, such as an infant, in accordance with at least one example embodiment of the inventive concept;
Fig. 2 illustrates in cross-section a device for a respiration arrangement according to at least one example embodiment of the inventive concept;
Fig. 3 illustrates in cross-section another device for a respiration arrangement according to at least one example embodiment of the inventive concept;
Fig. 4 illustrates in cross-section yet another device for a respiration arrangement according to at least one example embodiment of the inventive concept; and
Fig. 5A and 5B illustrate perspective views of the first and second flow guiding elements.

### Detailed Description of Example Embodiments

In the present detailed description, various embodiments of the inventive concept are mainly described with reference to a device for a respiration arrangement, and a respiration arrangement for breathing support to a patient, preferably an infant.

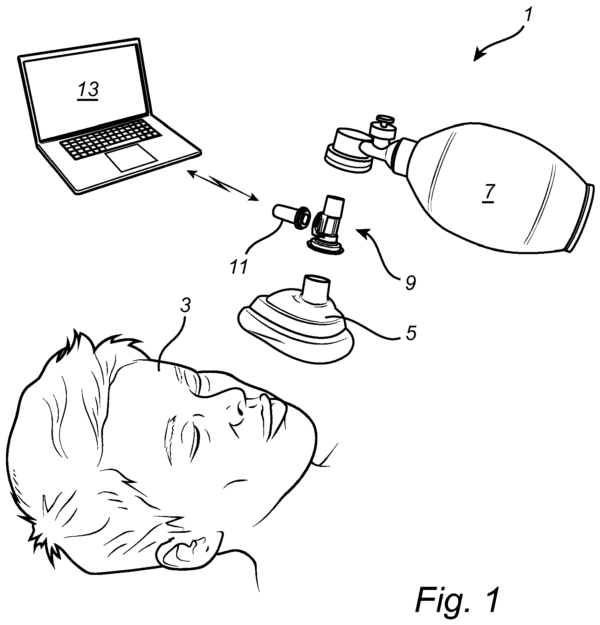

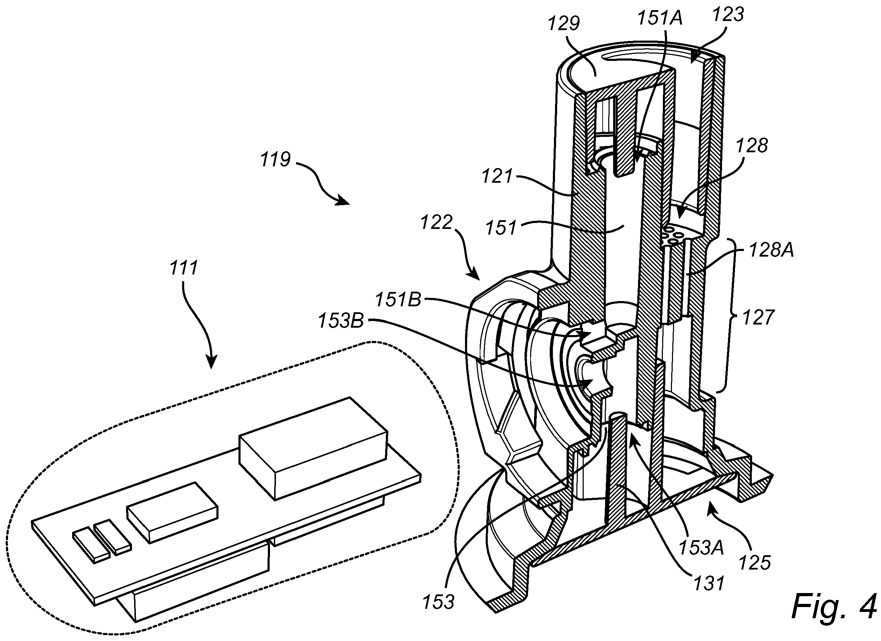

Fig. 1 is a schematic view illustrating a respiration arrangement 1 for breathing support to a patient 3, such as an infant 3. The respiration arrangement 1 comprises a face mask 5 adapted to fit over the mouth and nose of the patient 3, an air/gas source 7 providing air or gas to the face mask 5, and a device 9 providing an air/gas pathway from the air/gas source 7 to the face mask 5. Note that in Fig. 1, the device 9 is embodied as an airway adapter detachably connected to the face mask 5, but according to at least one example embodiment the face mask 5 is integrated with the airway adapter and thus forming a part of the device 9. The device 9 may e.g. be arranged with corresponding tubular portions in both ends of the device 9. That is, the end portion facing the face mask 5 may be tubular in a corresponding manner as the end portion facing the air/gas source, and thus not comprising the diverging portion presented in Fig. 1. The face mask 5 may be a separate/existing face mask or a soft mask, and/or the device 9 may form part of a hard mask connectable to a soft mask for forming an integrated device/face mask. The respiration arrangement 1 may further comprise a pressure sensor unit 11 detachably arranged to the device 9. According to at least one example embodiment of the inventive concept, the pressure sensor unit 11 is forming a part of the device 9. The pressure sensor unit 11 is typically used for measuring pressure generated by the fluid flowing through the device, which pressure can be used for calculating the fluid flow through the device, from the air/gas source 7 to the face mask 5, and thus the amount of air/gas fed to the patient 3 and/or calculating the fluid flow from the face mask 5 towards the air/gas source 7, i.e. the air/gas exhaled from the patient and transported in the other direction of the device 9. The pressure sensor unit 11 may for example comprise a differential pressure sensor, measuring the pressure drop over a flow constriction in the device (described in more detail with reference to Fig. 4). The respiration arrangement 1 may furthermore comprise a display 13, or another information presenting means, communicating with the pressure sensor unit 11. The display 13 is typically adapted to present the fluid flow through the device 9 and thus the amount of air/gas fed to the patient 3 and/or the amount of air/gas exhaled from the patient 3.

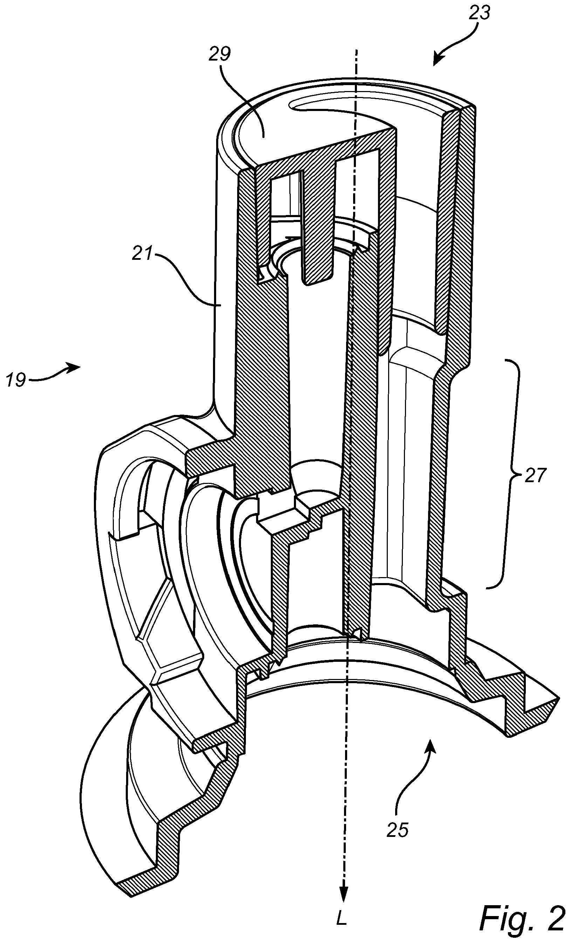

Figs. 2-4 illustrates various embodiments of the device 9 of Fig. 1. In Fig. 2, the device 19 is illustrated in cross-section along the longitudinal direction L of the device 19. The device 19 comprises a conduit 21 having a first opening 23 connectable to a resuscitation bag or other gas source (shown in Fig. 1), and a second opening 25 connectable to a face mask (also shown in Fig. 1). Hereby, a fluid pathway is established along a longitudinal direction L of the conduit 21, from the first opening 23 to the second opening 25, and naturally, a fluid pathway from the second opening 25 to the first opening 23.

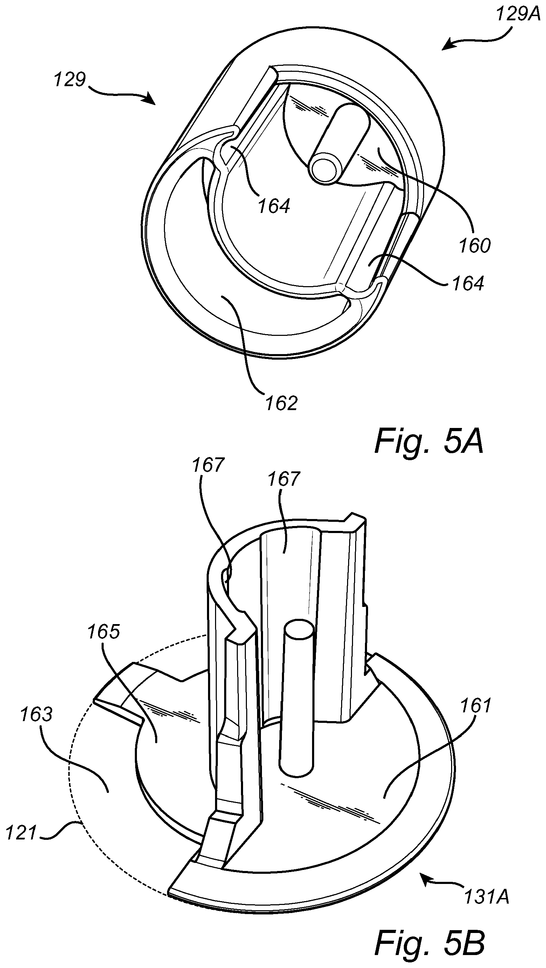

As seen in Fig. 2, the device 19 comprises a flow constriction 27, or flow restriction 27, in the conduit 21 which upon fluid flow through the conduit 21 results in a pressure difference over the flow constriction 27. The device 19 in Fig. 2 further comprises a flow guiding element 29 arranged in the conduit 21 between the flow constriction 27 and the first opening 23. The flow guiding element 29 is arranged to, upon fluid flow from the first opening 23 to the second opening 25, guide fluid flow to the flow constriction 27, which is described in more detail with reference to Fig. 5A. Hereby, any accuracy problem originating from the flow measurement with regards to asymmetrical geometry of the flow constriction 27, as e.g. flow constrictions not being centrally arranged in the conduit 21, or devices comprising other fluid ports (e.g. a pressure connecting port) risking by-pass of fluid or other disturbances (such as e.g. turbulent eddies), is mitigated. Thus, the flow guiding element 29 enables accurate measurement independently of the orientation of the incoming fluid flow to the device 19.

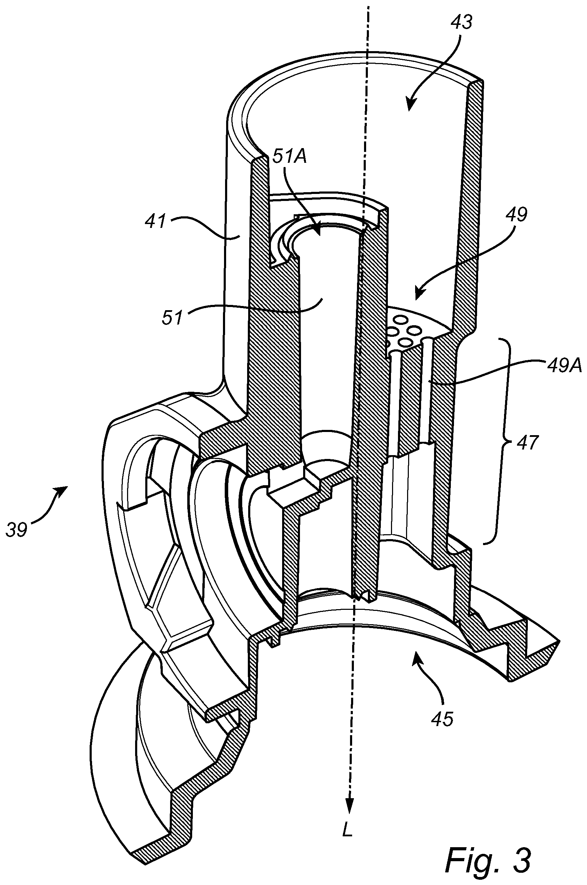

Fig. 3 illustrates a similar device 39 as the one shown in Fig. 2, but with the difference that there is no flow guiding element present in the device 39, and the flow constriction is designed differently. Thus, the device 39 comprises a conduit 41 having a first opening 43 connectable to a resuscitation bag or other gas source (shown in Fig. 1), and a second opening 45 connectable to a face mask (also shown in Fig. 1) whereby a fluid pathway is established along a longitudinal direction L of the conduit 41. In a similar manner as in Fig. 2, the flow constriction 47 in the conduit 41 of Fig. 3 is arranged to, upon fluid flow through the conduit, cause a pressure difference over the flow constriction 47. In Fig. 3, the flow constriction 47 comprises a laminar flow section 49, having a plurality of elongated channels 49A (only one of the channels is indicated in Fig. 3) arranged in the longitudinal direction L of the conduit 41.

The device 39 of Fig. 3 further comprises a pressure connecting port 51 arranged in pressurized communication with fluid between the flow constriction 47 and the first opening 43 (i.e. upstream of the flow constriction 47 when fluid flows from the first opening 43 to the second opening 45). The pressure connecting port 51 is arranged in the longitudinal direction L of the conduit 41. That is, the inlet 51A of the pressure connecting port 51 extends in a geometrical plane perpendicular to the longitudinal direction L of the conduit 41. Thus, the main direction of the pressure connecting port 51 is the same as the main direction of the plurality of elongated channels 49A. Hereby, manufacturing of the device 39 by e.g. injection molding is facilitated. Moreover, by having a pressure connecting port 51 with an inlet 51A facing the first opening 43 of the conduit, a filter, such as e.g. a bacteria filter, may easily be inserted into the device 39. However, it should be noted that according to at least one alternative embodiment of the inventive concept, the inlet 51A of the pressure connecting port 51 extends in a geometrical plane parallel to the longitudinal direction L of the conduit 41.

The pressure connecting port 51 is arranged and configured for being connected to a pressure sensor unit as shown in Fig. 4. Hereby, the pressure between the flow constriction 47 and the first opening 43 may be measured, which pressure can be used to determine the fluid flow through the conduit 41.

Fig. 4 illustrates a similar device 119 as those shown in Figs. 2 and 3. Thus, the device 119 comprises a conduit 121 having a first opening 123 connectable to a resuscitation bag or other gas source (shown in Fig. 1), and a second opening 125 connectable to a face mask (also shown in Fig. 1) whereby a fluid pathway is established along a longitudinal direction L of the conduit 121. In a similar manner as in Fig. 2, the flow constriction 127 in the conduit 121 of Fig. 4 is arranged to, upon fluid flow through the conduit 121, cause a pressure difference over the flow constriction 127. In Fig. 4, the flow constriction 127 comprises a laminar flow section 128, having a plurality of elongated channels 128A (only one of the channels is indicated in Fig. 4) arranged in the longitudinal direction L of the conduit 121, in a similar manner as in Fig. 3.

As seen in Fig. 4, the device 119 comprises a first pressure connecting port 151 arranged in pressurized communication with fluid between the flow constriction 127 and the first opening 123, and a second pressure connecting port 153 arranged in pressurized communication with fluid between the flow constriction 127 and the second opening 125 (i.e. downstream of the flow constriction 127 when fluid flows from the first opening 123 to the second opening 127). The first pressure connecting port 151 is similar to the pressure connecting port 51 described with reference to Fig. 2. Thus, the first pressure connecting port 151 is arranged in the longitudinal direction L. That is, the inlet 151A of the first pressure connecting port 151 extends in a geometrical plane perpendicular to the longitudinal direction L of the conduit 121, and the main direction of the first pressure connecting port 151 is the same as the main direction of the plurality of elongated channels 128A. In a corresponding manner, the second pressure connecting port 153 is arranged in the longitudinal direction L, but with an inlet 153A facing in an opposite direction to the inlet 151A of the first pressure connecting port 151. Thus, the inlet 153A of the second pressure connecting port 153 is facing the second opening 125 of the conduit 121. Hence, also the inlet 153A of the second pressure connecting port 153 extends in a geometrical plane perpendicular to the longitudinal direction L of the conduit 121.

Thus, the first and the second pressure connecting ports 151, 153 are arranged in the device 119 to provide ports for upstream and downstream pressure, relative the flow constriction 127. As seen in Fig. 4, a pressure sensor unit 111 is illustrated as detachably arranged to the conduit 121. In more detail, the conduit 121 comprises a pressure sensor connecting portion 122 arranged and configured for connection to the pressure sensor unit 111. In Fig. 4, the pressure sensor connecting portion 122 is tube shaped and extends from the conduit 121 perpendicular to the longitudinal direction L. Moreover, the first and second pressure connecting ports 151, 153 are arranged in the device 119 to have their respective outlet 151B, 153B in the pressure sensor connecting portion 122, wherein the respective outlet 151B, 153B is facing in a direction perpendicular relative the respective inlet 151A, 153A of the first and second pressure connecting ports 151, 153. That is, the respective outlet 151B, 153B of first and second pressure connecting ports 151, 153 has a cross section parallel to the longitudinal direction L. However, it should be noted that the inlets 151A, 153A of the first and second pressure connecting ports are not arranged in the conduit to receive any fluid flow, but rather to act as a port for enabling fluid communication enabling measurement of the pressure of the fluid upstream and downstream of the flow constriction. Correspondingly, the outlets 151B, 153B of the first and second pressure connecting ports 151, 153 are not arranged in the conduit 121 to discharge any fluid flow, but rather to act as a port for enabling fluid communication to the pressure sensor in the pressure sensor unit 111.

By providing the first and second pressure connecting ports 151, 153 arranged in the device 119 to provide ports for upstream and downstream pressure, relative the flow constriction 127, as shown in Fig. 4, the differential pressure over the flow constriction 127 can be measured by a differential pressure sensor comprised in the pressure sensor unit 111. The pressure sensor unit may e.g. comprise a battery, a CPU or processor, a memory, a wireless communication means, such as e.g. a Bluetooth module, and a battery. The pressure sensor unit 111 and flow constriction 127 may be referred to as a monitoring arrangement, which together with an information presenting means (as shown in Fig 1 as a display 13), are arranged to calculate and present the amount of air/gas flowing to/from the patient.

The device 119 in Fig. 4 comprises a first flow guiding element 129 arranged in the conduit 121 between the flow constriction 127 and the first opening 123 and configured to guide fluid flow from the first opening 123 towards the flow constriction 127 when fluid flows from the first opening 123 towards the second opening 125, and comprises a second flow guiding element 131 arranged in the conduit 121 between the flow constriction 127 and the second opening 125 and configured to guide fluid flow from the second opening 125 towards the flow constriction 127 when fluid flows from the second opening 125 towards the first opening 123. The first flow guiding element 129 corresponds in large to the flow guiding element 29 of the device 19 of Fig. 2. Thus, the first flow guiding element 129 is arranged and configured to guide fluid flow to the flow constriction 127 to mitigate any accuracy problem originating from the flow measurement with regards to asymmetrical geometry of the flow constriction 127, or as here, with regards to undesirable fluid flow into the first pressure connecting port 151. Thus, the first flow guiding element 129 enables accurate measurement independently of the orientation of the incoming fluid flow, via the first opening 123, to the device 119.

The second flow guiding element 131, is configured to guide fluid flow to the flow constriction 127 to mitigate any accuracy problem originating from the flow measurement with regards to asymmetrical geometry of the flow constriction 127, or as here, with regards to undesirable fluid flow into the second pressure connecting port 153. Thus, the second flow guiding element 131 enables accurate measurement independently of the orientation of the incoming fluid flow, via the second opening 125, to the device 119.

Fig. 5A and Fig. 5B illustrates detailed views of the first flow guiding element 129 and the second flow guiding element 131, respectively.

The first flow guiding element 129 comprises a first surface 129A facing the first opening 123 when being arranged in the conduit 121 of the device 119, wherein the first surface 129A comprising a shielding portion 160. The first flow guiding element 129 is arranged and configured in the conduit 121 such that the shielding portion 160 covers the first pressure connecting port 151, or at least the inlet 151A of the first pressure connecting port 151. Hereby, fluid flow into the first pressure connecting port 151 can be avoided by the shielding portion 160.

The first flow guiding element 129 further comprises a fluid passage 162 enabling fluid flow between the first opening 123 and the flow constriction 127 when being arranged in the conduit 121 of the device 119. The first flow guiding element 129 is arranged and configured in the conduit 121 such that the fluid passage 162 covers the flow constriction 127, or at least guides the fluid flow towards the flow constriction 127 when fluid flows through the device 119 from the first opening 123 to the second opening 125. In Fig. 5A, the fluid passage 162 is shaped to enable a minimum flow resistance by having a crescent shaped cross section (cross section being perpendicular to the longitudinal direction L of the conduit 121).

Moreover, in the embodiment of Fig. 5A, the first flow guiding element 129 comprises two pressure conduits 164 arranged in proximity to an end portion of the fluid passage 162 to provide fluid channels between the fluid passage 162 and the first pressure connecting port 151. The pressure conduits 164 enables the first pressure connecting port 151 to be in fluid communication, without receiving any fluid flow, with fluid present between the first opening 123 and the flow constriction 127. Thus, the pressure of the fluid can be measured by a pressure sensor connected to the first pressure connecting port 151.

As can be seen in Fig. 5B, the second flow guiding element 131 comprises a first surface 131A facing the second opening 125 when being arranged in the conduit 121 of the device 119, wherein the first surface 131A comprising a shielding portion 161. The second flow guiding element 131 is arranged and configured in the conduit 121 such that the shielding portion 161 covers the second pressure connecting port 153, or at least the inlet 153A of the second pressure connecting port 153. Hereby, fluid flow into the second pressure connecting port 153 can be avoided by the shielding portion 161.

The second flow guiding element 131 further comprises a fluid passage 163 enabling fluid flow between the second opening 125 and the flow constriction 127 when being arranged in the conduit 121 of the device 119. The second flow guiding element 131 is arranged and configured in the conduit 121 such that the fluid passage 163 covers at least a part of the flow constriction 127, or at least guides the fluid flow towards the flow constriction 127 when fluid flows through the device 119 from the second opening 125 to the first opening 123.

The second flow guiding element 131 further comprises a protecting portion 165 arranged and configured in the conduit 121 to cover at least a part of the flow constriction 127, and thereby preventing liquid, such as saliva or vomit, from the patient to reach into the device 119 beyond the second flow guiding element 131.

In Fig. 5B, the fluid passage 163 is arranged between internal walls of the conduit 121 and the protecting portion 165. In the embodiment in Fig. 5B, the fluid passage 163 of the second flow guiding element 131 is arranged in proximity to the internal walls of the conduit 121, and the protecting portion 165 is arranged closer to the center of the conduit 121. Herby, the protecting portion 165 can efficiently hinder any liquid expectorated from the patient.

Moreover, in the embodiment of Fig. 5B, the second flow guiding element 131 comprises two pressure conduits 167 arranged to provide fluid channels between fluid present between the second opening 125 and the flow constriction 127, and the second pressure connecting port 153. The pressure conduits 167 enables the second pressure connecting port 153 to be in fluid communication, without receiving any fluid flow, with fluid present between the second opening 125 and the flow constriction 127. Thus, the pressure of the fluid can be measured by a pressure sensor connected to the second pressure connecting port 153.

It should be noted that the air/gas source 7 may e.g. be an air source 7, or another type of gas source providing e.g. oxygen. In one example embodiment, the air/gas source 7 is a resuscitation bag/bellow 7 or a T-piece resuscitator.

Even though the inventive concept has been described with reference to specific exemplifying embodiments thereof, many different alterations, modifications and the like will become apparent for those skilled in the art. Also, it should be noted that parts of the pressure sensor unit 11, 111 may be omitted, interchanged or arranged in various ways, the device 9, 119 yet being able to perform the functionality of the present inventive concept. Moreover, the patient interface may instead of a face mask be an endotracheal tube, a laryngeal mask or nasal prongs.

## Claims

1. A device (119) for a respiration arrangement comprising:
- a conduit (121) having a first opening connectable to an air/gas source such as a resuscitation bag, and a second opening connectable to a patient interface, such that a fluid pathway along a longitudinal direction of the conduit is established from the first opening to the second opening;
- a flow constriction (127) arranged in the conduit which upon fluid flow through the conduit results in a pressure difference over the flow constriction, the flow constriction at least partly comprising a laminar flow section,
- a first pressure connecting port (151) arranged in pressurized communication with fluid between the flow constriction and the first opening, the first pressure connecting port being arranged in the longitudinal direction of the conduit and being connectable to a pressure sensor for measuring fluid pressure, ,
- a second pressure connecting port (153) arranged in pressurized communication with fluid between the flow constriction and the second opening, such that a differential pressure sensor can be connected to the first and second pressure connecting ports for measuring the differential pressure over the flow constriction,
**characterized in that**
the laminar flow section comprises a plurality of elongated channels arranged in the longitudinal direction of the conduit such that the first pressure connecting port and the plurality of elongated channels are arranged at least partly in parallel, and **in that** the device further
comprises a pressure sensor connecting portion (122) arranged between the first opening and the second opening, wherein the first and second pressure connecting ports are arranged in the device to have respective openings in the pressure sensor connecting portion, each of the openings of the first and second pressure connecting ports having a cross section parallel to the longitudinal direction.

2. The device according to claim 1, wherein the device is an adapter detachably arranged to the patient interface.

3. The device according to any one of the preceding claims, wherein the conduit is arranged to provide an air/gas pathway in a direction from the air/gas source to the patient interface via the first opening, the conduit and the second opening in that order, and in a direction from the patient interface to the air/gas source via the second opening, the conduit and the first opening in that order.

4. The device according to any one of the preceding claims, wherein a cross section of the conduit perpendicular to the longitudinal direction, at the flow constriction, comprises both the at least one pressure connecting port and the elongated channels such that at least a part of the pressure connecting port is sharing the same conduit space as the flow constriction.

5. The device according to any one of the preceding claims, wherein the laminar flow section fully accommodate the passageway in the flow constriction.

6. The device according to any one of the preceding claims, wherein the device is injected molded.

7. The device according to any one of the preceding claims, wherein the second pressure connecting port comprises an elongated fluid cavity having a longitudinal axis being parallel to the longitudinal direction of the conduit

8. The device according to any one of the preceding claims, wherein the pressure sensor connecting portion comprises a tube-shaped portion extending from the conduit perpendicular to the longitudinal direction.

9. The device according to any one of the preceding claims, wherein the openings of the first and second pressure connecting ports are arranged in the opposite end to inlet openings of the respective pressure connecting port which have a corresponding cross section perpendicular to the longitudinal direction of the conduit.

10. The device according to any one of the preceding claims, further comprising at least one flow guiding element arranged in the conduit between the first opening and the flow constriction, and configured to guide fluid flow to the flow constriction.

11. The device according to claim 10, wherein the flow constriction is asymmetrically arranged in the conduit, and the flow guiding element is arranged to direct the fluid flow towards the flow constriction.

12. The device according to any one of the preceding claims, further comprising a pressure sensor detachably arranged to the conduit for measuring pressure upstream and/or downstream of the flow constriction.

13. A respiration arrangement for breathing support to a patient, preferably an infant, comprising:
- a patient interface, such as a face mask adapted to fit over the mouth and nose of the patient,
- an air/gas source providing air or gas to the patient interface, and
- a device according to any one of claims 1-12.

## Patentansprüche

1. Vorrichtung (119) für eine Beatmungsanordnung, wobei die Vorrichtung Folgendes umfasst:
- eine Leitung (121), die eine erste Öffnung, die mit einer Luft-/Gasquelle, wie beispielsweise einem Wiederbelebungsbeutel, verbindbar ist, und eine zweite Öffnung, die mit einer Patientenverbindungsstelle verbindbar ist, aufweist, so dass eine Fluidleitungsbahn entlang einer Längsrichtung der Leitung von der ersten Öffnung bis zu der zweiten Öffnung hergestellt wird,
- eine Durchflusseinschränkung (127), die in der Leitung angeordnet ist, wobei sie auf einen Fluidstrom durch die Leitung zu einem Druckunterschied über die Durchflusseinschränkung führt, wobei die Durchflusseinschränkung zumindest teilweise eine Sektion mit laminarer Strömung umfasst,
- einen ersten Druckverbindungsanschluss (151), der in unter Druck gesetzter Verbindung mit Fluid zwischen der Durchflusseinschränkung und der ersten Öffnung angeordnet ist, wobei der erste Druckverbindungsanschluss in der Längsrichtung der Leitung angeordnet ist und mit einem Drucksensor zum Messen eines Fluiddrucks verbindbar ist,
- einen zweiten Druckverbindungsanschluss (153), der in unter Druck gesetzter Verbindung mit Fluid zwischen der Durchflusseinschränkung und der zweiten Öffnung angeordnet ist, so dass ein Differenzdrucksensor zum Messen des Differenzdrucks über die Durchflusseinschränkung mit dem ersten und dem zweiten Druckverbindungsanschluss verbunden werden kann,
**dadurch gekennzeichnet, dass**
die Sektion mit laminarer Strömung eine Vielzahl von länglichen Kanälen umfasst, die derart in der Längsrichtung der Leitung angeordnet sind, dass der erste Druckverbindungsanschluss und die Vielzahl von länglichen Kanälen zumindest teilweise parallel angeordnet sind, und
dadurch, dass die Vorrichtung ferner einen Drucksensor-Verbindungsabschnitt (122) umfasst, der zwischen der ersten Öffnung und der zweiten Öffnung angeordnet ist, wobei der erste und der zweite Druckverbindungsanschluss in der Vorrichtung so angeordnet sind, dass sie jeweilige Öffnungen in dem Drucksensor-Verbindungsabschnitt aufweisen, wobei jede der Öffnungen des ersten und des zweiten Druckverbindungsanschlusses einen Querschnitt, parallel zu der Längsrichtung, aufweist.

2. Vorrichtung nach Anspruch 1, wobei die Vorrichtung ein Adapter ist, der abnehmbar an der Patientenverbindungsstelle angeordnet ist.

3. Vorrichtung nach einem der vorhergehenden Ansprüche, wobei die Leitung so angeordnet ist, dass sie eine Luft- /Gasleitungsbahn in einer Richtung von der Luft-/Gasquelle zu der Patientenverbindungsstelle über die erste Öffnung, die Leitung und die zweite Öffnung in dieser Reihenfolge und in einer Richtung von der Patientenverbindungsstelle zu der Luft-/Gasquelle über die zweite Öffnung, die Leitung und die erste Öffnung in dieser Reihenfolge bereitstellt.

4. Vorrichtung nach einem der vorhergehenden Ansprüche, wobei ein Querschnitt der Leitung, senkrecht zu der Längsrichtung, an der Durchflusseinschränkung sowohl den mindestens einen Druckverbindungsanschluss als auch die länglichen Kanäle umfasst, so dass zumindest ein Teil des Druckverbindungsanschlusses denselben Leitungsraum wie die Durchflusseinschränkung gemeinsam benutzt.

5. Vorrichtung nach einem der vorhergehenden Ansprüche, wobei die Sektion mit laminarer Strömung den Durchgang in der Durchflusseinschränkung vollständig aufnimmt.

6. Vorrichtung nach einem der vorhergehenden Ansprüche, wobei die Vorrichtung spritzgegossen ist.

7. Vorrichtung nach einem der vorhergehenden Ansprüche, wobei der zweite Druckverbindungsanschluss einen länglichen Fluidhohlraum umfasst, der eine Längsachse aufweist, die parallel zu der Längsrichtung der Leitung ist.

8. Vorrichtung nach einem der vorhergehenden Ansprüche, wobei der Drucksensor-Verbindungsabschnitt einen röhrenförmigen Abschnitt umfasst, der sich von der Leitung aus senkrecht zu der Längsrichtung erstreckt.

9. Vorrichtung nach einem der vorhergehenden Ansprüche, wobei die Öffnungen des ersten und des zweiten Druckverbindungsanschlusses in dem Ende, entgegengesetzt zu Einlassöffnungen des jeweiligen Druckverbindungsanschlusses, angeordnet sind, die einen entsprechenden Querschnitt, senkrecht zu der Längsrichtung der Leitung, aufweisen.

10. Vorrichtung nach einem der vorhergehenden Ansprüche, die ferner mindestens ein Durchflussleitelement umfasst, das in der Leitung zwischen der ersten Öffnung und der Durchflusseinschränkung angeordnet und dafür konfiguriert ist, einen Fluidstrom zu der Durchflusseinschränkung zu leiten.

11. Vorrichtung nach Anspruch 10, wobei die Durchflusseinschränkung asymmetrisch in der Leitung angeordnet ist und das Durchflussleitelement dafür angeordnet ist, den Fluidstrom hin zu der Durchflusseinschränkung zu leiten.

12. Vorrichtung nach einem der vorhergehenden Ansprüche, die ferner einen Drucksensor umfasst, der zum Messen eines Drucks stromaufwärts und/oder stromabwärts von der Durchflusseinschränkung abnehmbar an der Leitung angeordnet ist.

13. Beatmungsanordnung zur Atmungsunterstützung für einen Patienten, vorzugsweise ein Kleinkind, die Folgendes umfasst:
- eine Patientenverbindungsstelle, wie beispielsweise eine Gesichtsmaske, die geeignet ist, über den Mund und die Nase des Patienten zu passen,
- eine Luft-/Gasquelle, die Luft oder Gas an die Patientenverbindungsstelle liefert, und
- eine Vorrichtung nach einem der Ansprüche 1 bis 12.

## Revendications

1. Dispositif (119) pour un agencement de respiration comprenant :
- un conduit (121) ayant une première ouverture pouvant être connectée à une source d'air/de gaz, telle qu'un ballon de réanimation, et une seconde ouverture pouvant être connectée à une interface patient, de manière à ce qu'un chemin de fluide soit établi le long d'une direction longitudinale du conduit entre la première ouverture et la seconde ouverture ;
- un étranglement de flux (127) disposé dans le conduit ce qui, lors du passage d'un fluide dans le conduit, entraîne une différence de pression au niveau de l'étranglement de flux, l'étranglement de flux comprenant au moins partiellement une section de flux laminaire,
- un premier raccord de pression (151) disposé en communication pressurisée avec du fluide entre l'étranglement de flux et la première ouverture, le premier raccord de pression étant disposé dans la direction longitudinale du conduit et pouvant être connecté à un capteur de pression pour mesurer la pression du fluide,
- un second raccord de pression (153) disposé en communication pressurisée avec du fluide entre l'étranglement de flux et la seconde ouverture, de manière à ce qu'un capteur de pression différentielle puisse être connecté au premier et second raccord de pression afin de mesurer la pression différentielle au niveau de l'étranglement de flux,
**caractérisé en ce que**
la section de flux laminaire comprend une pluralité de canaux allongés disposés dans la direction longitudinale du conduit de manière à ce que le premier raccord de pression et la pluralité de canaux allongés soient au moins partiellement parallèles, et
**en ce que** le dispositif comprend en outre une portion de raccordement de capteur de pression (122) disposée entre la première ouverture et la seconde ouverture, dans lequel le premier et le second raccord de pression sont disposés dans le dispositif pour avoir des ouvertures respectives dans la portion de raccordement de capteur de pression, chacune des ouvertures du premier et du second raccord de pression ayant une section transversale parallèle à la direction longitudinale.

2. Dispositif selon la revendication 1, dans lequel le dispositif est un adaptateur disposé de manière détachable sur l'interface patient.

3. Dispositif selon l'une quelconque des revendications précédentes, dans lequel le conduit est disposé pour fournir un chemin d'air/de gaz dans une direction allant de la source d'air/de gaz à l'interface patient à travers la première ouverture, le conduit et la seconde ouverture dans cet ordre, et dans une direction de l'interface patient à la source d'air/de gaz à travers la seconde ouverture, le conduit et la première ouverture dans cet ordre.

4. Dispositif selon l'une quelconque des revendications précédentes, dans lequel une section transversale du conduit perpendiculaire à la direction longitudinale, au niveau de l'étranglement de flux, comprend aussi bien l'au moins un raccord de pression et les canaux allongés de manière à ce qu'au moins une partie du raccord de pression partage le même espace de conduit que l'étranglement de flux.

5. Dispositif selon l'une quelconque des revendications précédentes, dans lequel la section de flux laminaire s'adapte parfaitement au passage dans l'étranglement de flux.

6. Dispositif selon l'une quelconque des revendications précédentes, dans lequel le dispositif est moulé par injection.

7. Dispositif selon l'une quelconque des revendications précédentes, dans lequel le second raccord de pression comprend une cavité de fluide allongée ayant un axe longitudinal parallèle à la direction longitudinale du conduit.

8. Dispositif selon l'une quelconque des revendications précédentes, dans lequel la portion de raccordement du capteur de pression comprend une portion en forme de tube s'étendant du conduit perpendiculairement à la direction longitudinale.

9. Dispositif selon l'une quelconque des revendications précédentes, dans lequel les ouvertures du premier et du second raccord de pression sont disposées dans l'extrémité opposée à des ouvertures d'entrée du raccord de pression respectif, qui ont une section transversale correspondante perpendiculaire à la direction longitudinale du conduit.

10. Dispositif selon l'une quelconque des revendications précédentes, comprenant en outre au moins un élément de guidage du flux disposé dans le conduit entre la première ouverture et l'étranglement de flux, et configuré pour guider le flux de fluide vers l'étranglement de flux.

11. Dispositif selon la revendication 10, dans lequel l'étranglement de flux est disposé de manière asymétrique dans le conduit, et l'élément de guidage du flux est disposé pour diriger le flux de fluide vers l'étranglement de flux.

12. Dispositif selon l'une quelconque des revendications précédentes, comprenant en outre un capteur de pression disposé de manière détachable au niveau du conduit pour mesurer la pression en amont et/ou en aval de l'étranglement de flux.

13. Agencement de respiration pour assistance respiratoire à un patient, de préférence un nourrisson, comprenant :
- une interface patient, telle qu'un masque facial adapté pour s'ajuster sur la bouche et le nez du patient,
- une source d'air/de gaz fournissant de l'air ou du gaz à l'interface patient, et
- un dispositif selon l'une quelconque des revendications 1-12.
